# EUROPEAN PATENT APPLICATION

(11) **EP 4 600 645 A1**
(43) Date of publication of application: **13.08.2025**
(21) Application number: 24305228.9
(22) Date of filing: 12.02.2024
(51) Int. Cl.: G01N 33/50, G01N 33/564

(54) **METHOD TO DETERMINE THE RESPONSE TO RITUXIMAB TREATMENT IN A PATIENT SUFFERING FROM A PEMPHIGUS VULGARIS**

(71) Applicant: Institut National de la Santé et de la Recherche Médicale, 75013 Paris (FR); Université Paul Sabatier Toulouse III, 31400 Toulouse (FR); Centre National de la Recherche Scientifique, 75016 Paris (FR)
(72) Inventor: The designation of the inventor has not yet been filed
(74) Representative: Inserm Transfert

(57) **Abstract**

The present invention relates to the determination of relapsing in a patient suffering from a Pemphigus vulgaris after rituximab treatment.

Given the disappearance of autoreactive B cells after RTX treatment in PV patients, which correlated with the rapid decrease of Dsg-3-specific Tfh cells, and given the potential regulatory role of Tfr cells, the inventors assessed the re-emergence and long-term evolution of circulating autoreactive Dsg-3-Tfh and Tfr cells after RTX treatment and correlated these findings with the clinical and serological course of PV patients. Notably, the inventor determined that in PV patients before RTX treatment they were no dsg3-sp Tfr cells in their blood and that after RTX treatment and in patient with no relapse, dsg3-sp Tfr cells begin to appear in a high quantity.

Thus, the present invention relates to a method of determining whether a patient suffering from a Pemphigus vulgaris (PV) has or is at risk of relapsing after a rituximab (RTX) treatment.

## Description

### FIELD OF THE INVENTION:

The present invention relates to a method of determining whether a patient suffering from a Pemphigus vulgaris (PV) has or is at risk of relapsing after a rituximab (RTX) treatment.

### BACKGROUND OF THE INVENTION:

Pemphigus vulgaris (PV) is a life-threatening autoimmune disease of the skin and mucosae due to the production of antibodies (Abs) directed against the desmosomal protein desmoglein 3 (Dsg-3), an adhesion molecule of stratified epithelia (1,2). The inventors reported that first-line treatment with rituximab (RTX), the anti-CD20 mAb, allowed a large number of PV patients to achieve long-lasting remission off RTX and corticosteroids (CS) therapy (3). Interestingly, a large population of PV patients express HLA-DRB1*0402 which allows mechanistic and pathophysiological studies using HLA-DRB1*0402 tetramer. Notably, to better understand the immunological mechanisms that mediate long-lasting clinical remission after RTX treatment, the inventors recently showed, using peptide-HLA-DRB 1*0402 tetramer, that blood Dsg-3-specific CXCR5+ T follicular cells decreased rapidly after RTX, correlating with a sustained depletion of IgG-switched memory autoreactive B cells, leading to the disappearance of anti-Dsg-3 Ab-secreting cells (ASC) (4). Moreover, other studies suggested the presence of effective Tfh cells in damaged skin from PV patients (5-7). Finally, it has also been shown that both mouse Dsg3-specific Tfh cells and human blood Tfh cells expressing the inducible costimulator ICOS correlated positively with the anti-Dsg3 antibody response in PV (8).

Maturation of B cell responses takes place in germinal centers (GCs) of secondary lymphoid organs where a progressive increase of the Ab affinity occurs through somatic hypermutation. Eventually, only the fittest B cells capture the antigen (Ag) via high-affinity surface Ab, process and present it through MHCII molecules. B cell maturation in GCs is regulated by T follicular cells that express the chemokine receptor CXCR5. On one hand, T follicular helper cells (Tfh), through cognate TCR-MHC interactions, co-stimulation molecules and cytokines support B cell maturation (9,10). On the other hand, follicular regulatory CD4+ T (Tfr) cells, a population of regulatory T cells (Treg) that express the transcription factors BCL6 and FOXP3, colocalize in the GCs (11-13). There, Tfr cells control autoimmunity by suppressing excessive immune responses through, among others, the inhibitory coreceptor CTLA-4 (14,15). However, there is a discrepancy between some studies that suggested that Tfr cells inhibit and restrain overall GCs responses, including Ag-specific B cell responses (16,17) and other studies that did not evidence any significant effect of Tfr cells on Ag-specific Ab responses (18,19).

### SUMMARY OF THE INVENTION:

Given the disappearance of autoreactive B cells after RTX treatment in PV patients, which correlated with the rapid decrease of Dsg-3-specific Tfh cells, and given the potential regulatory role of Tfr cells, the inventors assessed the re-emergence and long-term evolution of circulating autoreactive Dsg-3-Tfh and Tfr cells after RTX treatment and correlated these findings with the clinical and serological course of PV patients. Notably, the inventor determined that in PV patients before RTX treatment they were no dsg3-sp Tfr cells in their blood and that after RTX treatment and in patient with no relapse, dsg3-sp Tfr cells begin to appear in a high quantity.

Thus, the present invention relates to a method of determining whether a patient suffering from a Pemphigus vulgaris (PV) has or is at risk of relapsing after a rituximab (RTX) treatment comprising the steps of: i) determining in a sample obtained from said patient the quantity of Dsg-3-Tfr cells ii) comparing said quantity determined at step i) with a predetermined reference value and iii) providing that a high quantity of Dsg-3-Tfr cells compared to said predetermined reference value is indicative of whether said patient has not or is not at risk of relapsing and providing that absence or a low quantity of Dsg-3-Tfr cells compared to said predetermined reference value is indicative of whether said patient has or is at risk of relapsing.

Particularly, the invention is defined by its claims.

### DETAILED DESCRIPTION OF THE INVENTION:

A first aspect of the invention relates to a method of determining whether a patient suffering from a Pemphigus vulgaris (PV) has or is at risk of relapsing after a rituximab (RTX) treatment comprising the steps of: i) determining in a sample obtained from said patient the quantity of Dsg-3-Tfr cells ii) comparing said quantity determined at step i) with a predetermined reference value and iii) providing that a high quantity of Dsg-3-Tfr cells compared to said predetermined reference value is indicative of whether said patient has not or is not at risk of relapsing and providing that absence or a low quantity of Dsg-3-Tfr cells compared to said predetermined reference value is indicative of whether said patient has or is at risk of relapsing.

In other word, the invention relates to a method of determining whether a patient suffering from a Pemphigus vulgaris (PV) will response to a rituximab (RTX) treatment comprising the steps of: i) determining in a sample obtained from said patient the quantity of Dsg-3-Tfr cells ii) comparing said quantity determined at step i) with a predetermined reference value and iii) providing that a high quantity of Dsg-3-Tfr cells compared to said predetermined reference value is indicative of whether said patient response positively to the RTX treatment and that absence or a low quantity of Dsg-3-Tfr cells compared to said predetermined reference value is indicative of whether said patient not response positively to the RTX treatment.

As used herein, the term "relapse" denotes that a patient return to a previous bad condition or a worse way of life or suffers again of the same disease after making an improvement. According to the invention, a patient suffering from a Pemphigus vulgaris (PV) will relapse after a RTX treatment he received initially depending of the quantity of Dsg-3-Tfr cells.

As used herein, the term "response positively to the RTX treatment" denotes that the patient will not relapse after he received the RTX treatment.

According to the invention, when the patient is not relapsing, that's means that he is in full remission.

In a particular embodiment, the Dsg-3-Tfr cells are autoreactive Dsg-3-Tfr cells.

In another particular embodiment, the Dsg-3-Tfr cells are circulating autoreactive Dsg-3-Tfr cells.

In another particular embodiment, the Dsg-3-Tfr cells overexpress the markers PD1, ICOS, and CD25.

In another particular embodiment, the Dsg-3-Tfh cells are polarized towards Tfh1* and Tfh17 cells.

In a particular embodiment, the determination of the quantity of Dsg-3-Tfr cells or Dsg-3-Tfh cells is realized between 6 months and 90 months after the beginning of the treatment with RTX.

Another aspect of the invention relates to a method of determining whether a patient suffering from a Pemphigus vulgaris (PV) has or is at risk of relapsing after a rituximab (RTX) treatment comprising the steps of: i) determining in a sample obtained from the patient the quantity of Dsg-3-Tfh cells ii) comparing said quantity determined at step i) with a predetermined reference value and iii) providing that a low quantity of Dsg-3-Tfh cells compared to said predetermined reference value is indicative of whether a patient has not or is not at risk of relapsing and that a high quantity of Dsg-3-Tfh cells compared to said predetermined reference value is indicative of whether a patient has or is at risk of relapsing.

According to the invention, the Tfr/Tfh ratio can also be calculated to determine if a patient with Pemphigus vulgaris will relapse after RTX treatment.

Thus, a 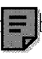 her aspect of the invention relates to a method of determining whether a patient suffering from a Pemphigus vulgaris (PV) has or is at risk of relapsing after a rituximab (RTX) treatment comprising the steps of: i) determining in a sample obtained from the patient the quantity of Dsg-3-Tfr cells and Dsg-3-Tfh cells ii) calculating the Tfr/Tfh ratio and iii) providing that a ratio < 1 is indicative of whether a patient has or is at risk of relapsing and that a ratio > or = 1 is indicative of whether a patient has not or is not at risk of relapsing.

According to the invention, the sample can be blood and peripheral-blood.

As used herein, the term "patient" denotes a mammal, such as a rodent, a feline, a canine, and a primate. Particularly, the patient according to the invention is a human and more particularly, the patient is a human suffering from a Pemphigus vulgaris according to the invention.

As used herein, the term "follicular helper T lymphocytes" or "Tfh" 'denotes antigen-experienced CD4+ T cells found in the periphery within B cell follicles of secondary lymphoid organs such as lymph nodes, spleen and Peyer's patches, and are identified by their constitutive expression of the B cell follicle homing receptor CXCR5. Upon cellular interaction and cross-signaling with their cognate follicular (Fo B) B cells, Tfh cells trigger the formation and maintenance of germinal centers through the expression of CD40 ligand (CD40L) and the secretion of IL-21 and IL-4. Tfh cells also migrate from T cell zones into these seeded germinal centers, predominantly composed of rapidly dividing B cells mutating their Ig genes. Within germinal centers, Tfh cells play a critical role in mediating the selection and survival of B cells that go on to differentiate either into long-lived plasma cells capable of producing high affinity antibodies against foreign antigen, or germinal center-dependent memory B cells capable of quick immune re-activation in the future if ever the same antigen is re-encountered. Tfh cells are also thought to facilitate negative selection of potentially autoimmune-causing mutated B cells in the germinal center. However, the biomechanisms by which Tfh cells mediate germinal center tolerance are yet to be fully understood. Notably, Tfh cells from second lymphoid organs can exit these organs and join the blood circulation, where they correlate with Tfh activity in second lymphoid organs and, therefore, can serve as a mirror of Tfh cell activity without sampling second lymphoid organs but the blood only. These blood Tfh cells are named circulating Tfh cells.

As used herein, the term "Follicular regulatory T cell" or "Tfr" 'denotes a special subgroup of regulatory T (Treg) cells. Tfr cells play an important role in regulating the germinal center (GC) response, especially modulating follicular helper T (Tfh) cells and GC-B cells, thereby affecting the production of antibodies. Similar to their Tfh cell counterpart, these cells can exit second lymphoid organs to join the blood circulation.

As used herein, the terms "Dsg-3-specific Tfh cells" or "Dsg-3 specific Tfr cells" denote Tfh or Tfr cells specific for the antigen demsoglein 3 (Dsg-3) that is to say which recognize said antigen.

As used herein, the terms "desmoglein 3 also known as Dsg-3 denotes a protein that in humans is encoded by the DSG3 gene. In the skin epidermis Desmoglein-3 is expressed in the basal lower layers of the epidermis, and dominates in terms of expression on mucosal surfaces compared to Desmoglein-1.

As used herein, the term "Pemphigus Vulgaris 'denotes a rare group of blistering autoimmune diseases that affect the skin and mucous membranes. In pemphigus, autoantibodies form against desmoglein, which forms the "glue" that attaches adjacent epidermal cells via attachment points called desmosomes. When autoantibodies attack desmogleins, the cells become separated from each other and the epidermis becomes detached, a phenomenon called acantholysis. This causes blisters that slough off and turn into sores. In some cases, these blisters can cover a large area of the skin.

### Determination of the quantity of the cells of the invention (Dsg-3-specific Tfh and Tfr cells:

As used herein, the term "quantity" refers to the quantity (cell by liter) or frequency of Dsg-3-specific Tfh and Tfr cells notably in the blood. Particularly, the term "frequency" refers to the percentage of Dsg-3-specific Tfh and Tfr cells compared to the total of T lymphocytes. Typically, the quantity or frequency of Dsg-3-specific Tfh and Tfr cells may be determined by any technology known by a person skilled in the art. In some embodiments, the expression of the phenotypic marker is assessed at the mRNA level. Methods for assessing the transcription level of a molecule are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like. In some embodiments, the expression of the phenotypic marker is assessed at the protein level. Methods for determining a protein level in a sample are well-known in the art. Examples of such methods include, but are not limited to, immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

In some embodiment, the sample of the present invention (i.e., blood sample) is analyzed by flow cytometry to determine the quantity or frequency of lymphoid cells.

As used herein, the term "flow cytometry methods" refers to a technique for counting cells of interest, by suspending them in a stream of fluid and passing them through an electronic detection apparatus. Flow cytometry methods allow simultaneous multiparametric analysis of the physical and/or chemical parameters of up to thousands of particles per second, such as fluorescent parameters. Modern flow cytometry instruments usually have multiple lasers and fluorescence detectors. A common variation of flow cytometry techniques is to physically sort particles based on their properties, so as to purify or detect populations of interest, using "fluorescence-activated cell sorting".

As used herein, "fluorescence-activated cell sorting" (FACS) refers to a flow cytometric method for sorting a heterogeneous mixture of cells from a biological sample into two or more containers, one cell at a time, based upon the specific light scattering and fluorescent characteristics of each cell and provides fast, objective and quantitative recording of fluorescent signals from individual cells as well as physical separation of cells of particular interest. Accordingly, FACS can be used with the methods described herein to isolate and detect the subpopulation of monocytes cells.

In some embodiments, the preferred agents are antibodies that specifically bind the cell-surface markers, and can include polyclonal and monoclonal antibodies, and antigen-binding derivatives or fragments thereof. Well-known antigen binding fragments include, for example, single domain antibodies (dAbs; which consist essentially of single VL or VH antibody domains), Fv fragment, including single chain Fv fragment (scFv), Fab fragment, and F(ab')2 fragment. Methods for the construction of such antibody molecules are well known in the art. Accordingly, as used herein, the term "antibody" refers to an intact immunoglobulin or to a monoclonal or polyclonal antigen-binding fragment with the Fc (crystallizable fragment) region or FcRn binding fragment of the Fc region. Antigen-binding fragments may be produced by recombinant DNA techniques or by enzymatic or chemical cleavage of intact antibodies. "Antigen-binding fragments" include, inter alia, Fab, Fab', F(ab')2, Fv, dAb, and complementarity determining region (CDR) fragments, single -chain antibodies (scFv), single domain antibodies, chimeric antibodies, diabodies and polypeptides that contain at least a portion of an immunoglobulin that is sufficient to confer specific antigen binding to the polypeptide. The terms Fab, Fc, pFc', F(ab') 2 and Fv are employed with standard immunological meanings (Roitt, I. (1991) Essential Immunology, 7th Ed., (Blackwell Scientific Publications, Oxford)]. Such antibodies or antigen-binding fragments are available commercially from vendors such as R&D Systems, BD Biosciences, e-Biosciences, Proimmune and Miltenyi, or can be raised against these cell-surface markers by methods known to those skilled in the art.

In some embodiments, an agent that specifically bind to a cell-surface marker, such as an antibody or antigen-binding fragment, is labelled with a tag to facilitate the isolation and detection of the cell populations of the invention.

As used herein, the terms "label" or "tag" refer to a composition capable of producing a detectable signal indicative of the presence of a target, such as, the presence of a specific cell-surface marker in a biological sample. Suitable labels include fluorescent molecules, radioisotopes, nucleotide chromophores, enzymes, substrates, chemiluminescent moieties, magnetic particles, bioluminescent moieties, and the like. As such, a label is any composition detectable by spectroscopic, photochemical, biochemical, immunochemical, electrical, optical or chemical means needed for the methods to isolate and detect the cell populations of the invention. Non-limiting examples of fluorescent labels or tags for labeling the agents such as antibodies for use in the methods of invention include

Hydroxycoumarin, Succinimidyl ester, Aminocoumarin, Succinimidyl ester, Methoxycoumarin, Succinimidyl ester, Cascade Blue, Hydrazide, Pacific Blue, Maleimide, Pacific Orange, Lucifer yellow, NBD, NBD-X, R-Phycoerythrin (PE), a PE-Cy5 conjugate (Cychrome, R670, Tri-Color, Quantum Red), a PE-Cy7 conjugate, Red 613, PE-Texas Red, PerCP, Peridinin chlorphyll protein, TruRed (PerCP-Cy5.5 conjugate), FluorX, Fluoresceinisothyocyanate (FITC), BODIPY-FL, TRITC, X-Rhodamine (XRITC), Lissamine Rhodamine B, Texas Red, Allophycocyanin (APC), an APC-Cy7 conjugate, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, Alexa Fluor 790, Cy2, Cy3, Cy3B, Cy3.5, Cy5, Cy5.5 or Cy7.

According to the invention, an HLA-DRB1-0402 tetramer loaded with the Dsg-3 immunodominant peptide can be used to determine the quantity of the cells of the invention.

### Predetermined reference value

Predetermined reference value used for comparison of the quantity of the cells of the invention (Dsg-3-specific Tfh and Tfr cells) may comprise "cut-off" or "threshold" values that may be determined as described herein. Each reference ("cut-off") value for the cells of the invention may be predetermined by carrying out a method comprising the steps of:
a) providing a collection of samples from patients suffering of a Phemphigus vulgaris;
b) determining the quantity of the cells of the invention for each sample contained in the collection provided at step a);
c) ranking the samples according to said level
d) classifying said samples in pairs of subsets of increasing, respectively decreasing, number of members ranked according to their expression level,
e) providing, for each sample provided at step a), information relating to the presence of a relapsing or not after RTX treatment;
f) for each pair of subsets of samples, obtaining a Kaplan Meier curve of relapsing after to RTX treatment;
g) for each pair of subsets of samples calculating the statistical significance (p value) between both subsets
h) selecting as reference value for the level, the value of level for which the p value is the smallest.

For example the quantity of the cells of the invention has been assessed for 100 Pemphigus vulgaris samples of 100 patients. The 100 samples are ranked according to the quantity of the cells of the invention. Sample 1 has the best quantity of cells and sample 100 has the worst quantity of cells. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual presence or not of relapsing after RTX treatment, Kaplan Meier curves are prepared for each of the 99 groups of two subsets. Also for each of the 99 groups, the p value between both subsets was calculated.

The reference value is selected such as the discrimination based on the criterion of the minimum p value is the strongest. In other terms, the quantity of cells corresponding to the boundary between both subsets for which the p value is minimum is considered as the reference value. It should be noted that the reference value is not necessarily the median value of expression levels.

In routine work, the reference value (cut-off value) may be used in the present method to discriminate samples and therefore the corresponding patients.

Kaplan-Meier curves of percentage of relapsing after RTX treatment as a function of time are commonly used to measure the fraction of patients which response to the treatment and are well known by the man skilled in the art.

The man skilled in the art also understands that the same technique of assessment of the expression level of a protein should of course be used for obtaining the reference value and thereafter for assessment of the expression level of a protein of a patient subjected to the method of the invention. Particularly, other methods like median thresholding", "max-stat" and "Youden's J statistic can be used to determine a threshold.

### Therapeutics applications

In a further aspect, the invention also relates to a method for treating a Pemphigus vulgaris in a patient which is relapsing or will relapse after a RTX treatment as determined with the methods of the invention comprising the administration to said patient of corticosteroid treatment.

As used herein, the term "corticosteroid treatment" denotes a class synthetic analogues of steroids hormones. Two main classes of corticosteroids exist: glucocorticoids and mineralocorticoids.

The invention also relates to a pharmaceutical composition comprising a corticosteroid treatment for use in the treatment of a Pemphigus vulgaris in a patient which is relapsing or will relapse after a RTX treatment as described above.

Any therapeutic agent of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, intranasal, parenteral, intraocular, intravenous, intramuscular, intrathecal or subcutaneous administration and the like.

Particularly, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

In addition, other pharmaceutically acceptable forms include, e.g. tablets or other solids for oral administration; time release capsules; and any other form currently can be used.

Typically the corticosteroid treatment according to the invention are administered to the subject in a therapeutically effective amount.

By a "therapeutically effective amount" the corticosteroid treatment is meant a sufficient amount of the corticosteroid treatment for treating a Phemphigus vulgaris at a reasonable benefit/risk ratio applicable to any medical treatment. It will be understood, however, that the total daily usage of the corticosteroid treatment will be decided by the attending physician within the scope of sound medical judgment. The specific therapeutically effective dose level for any particular subject will depend upon a variety of factors including the disorder being treated and the severity of the disorder; activity of (the specific) the corticosteroid treatment employed; the specific composition employed, the age, body weight, general health, sex and diet of the subject; the time of administration, route of administration, and rate of excretion of the corticosteroid treatment employed; the duration of the treatment; drugs used in combination or coincidental with the corticosteroid treatment employed; and like factors well known in the medical arts. For example, it is well within the skill of the art to start doses of the corticosteroid treatment at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the corticosteroid treatment for the symptomatic adjustment of the dosage to the subject to be treated. A medicament typically contains from about 0.01 mg to about 500 mg of the corticosteroid treatment, preferably from 1 mg to about 100 mg of corticosteroid treatment. An effective amount of the drug is ordinarily supplied at a dosage level from 0.0002 mg/kg to about 20 mg/kg of body weight per day, especially from about 0.001 mg/kg to 7 mg/kg of body weight per day.

In a particular embodiment, the corticosteroid treatment may be used in a concentration between 0.01 µM and 20 µM, particularly, the anti-treatment may be used in a concentration of 0.01, 0.05, 0.1, 0.5, 1.0, 2.5, 5.0, 10.0, 15.0, 20.0 µM.

According to the invention, the corticosteroid treatment is administered to the subject in the form of a pharmaceutical composition.

Typically, the corticosteroid treatment may be combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising corticosteroid treatment as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The corticosteroid treatment of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized agent of the present inventions into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the corticosteroid treatment of the invention plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

The invention will be further illustrated by the following figures and examples. However, these examples and figures should not be interpreted in any way as limiting the scope of the present invention.

### FIGURES:

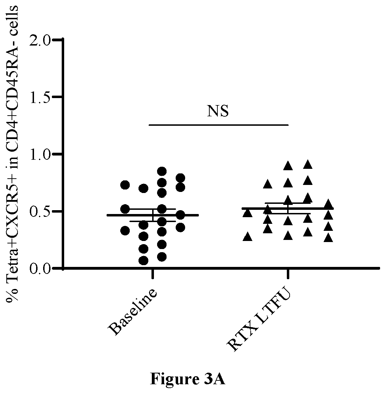

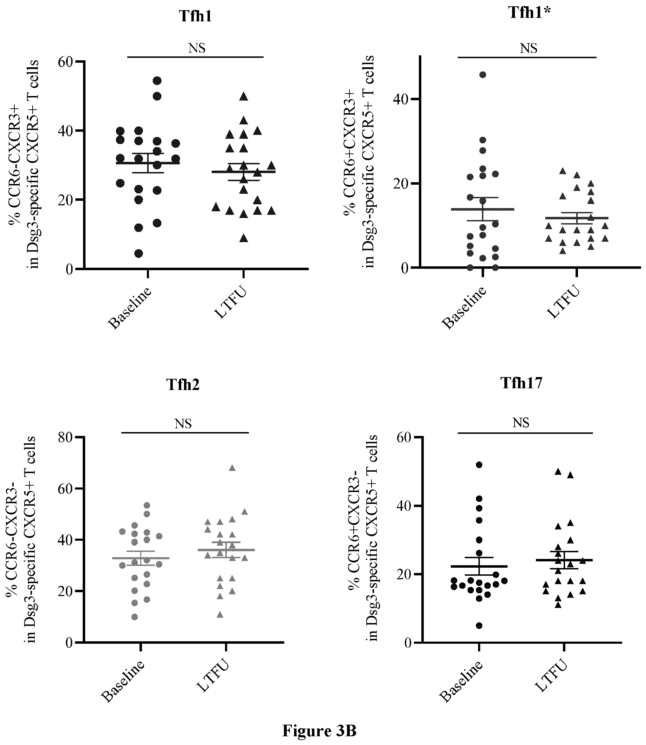

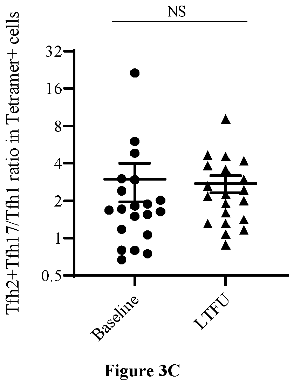

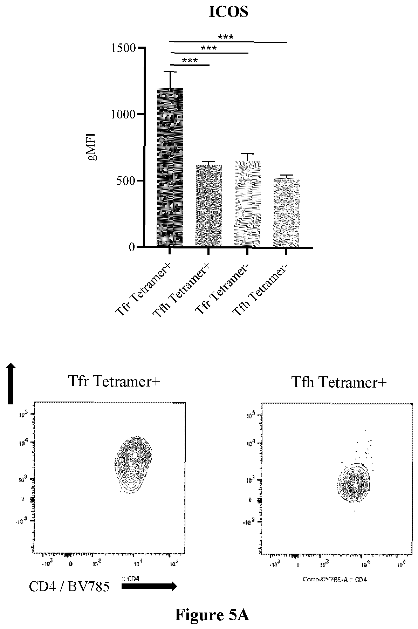

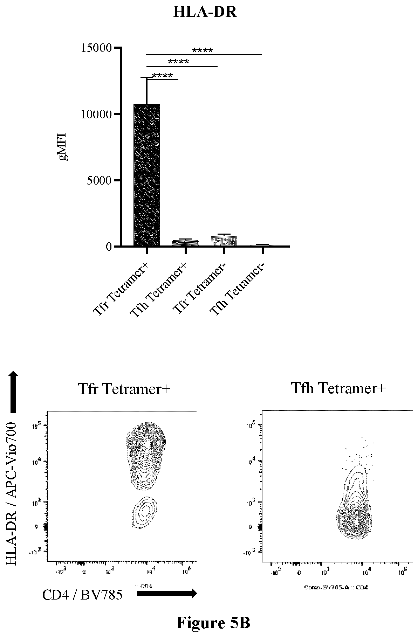

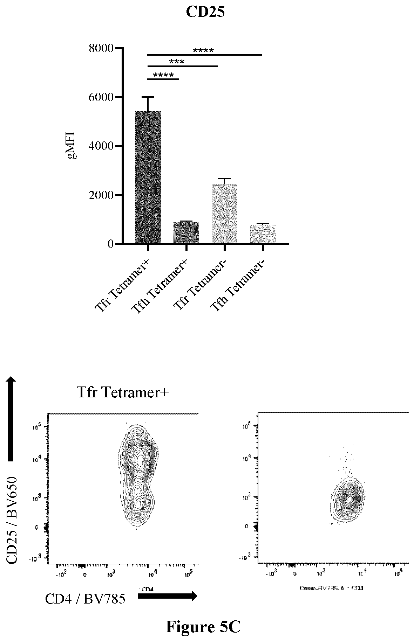

**Figure 1****: Flow chart of studied samples from PV Patients.**
**Figure 2****: Baseline polarization of circulating HLA-DRB0402 Dsg-3+ Tfh cells in PV** patients.
   **A)** Flow cytometry analysis of autoreactive Ag-experienced T cells (CD4+CD45RA-Tetramer+) using the tetramer HLA-DRB1*0402 loaded with human CLIP 87-101 or the immunodominant peptide of Dsg3 (190-204).
   **B)** Flow cytometry analysis of follicular autoreactive Ag-experienced T cells (CD4+CD45RA-Tetramer+CXCR5+).
   **C)** Strategy of assessment for CD4+CD45RA-Tetramer+CXCR5+ Tfh cell polarisation according to the expression of the chemokine receptors CXCR3 and CCR6.
   **D)** Frequencies of Tfu1 (red), Tfu1* (green), Tfh2 (pink) and Tfu17 (blue) cells in Ag-specific (Tetramer+) Tfh cells from PV patients (n= 20, triangular symbols) compared to non-Ag-specific (Tetramer-) Tfh cells from the same PV patients (square symbols) and HDs (n=20, circle columns).
   *P < 0.05; **P < 0.01
**Figure 3****: Evolution of circulating Dsg-3-specific Tfh cells in PV patients after RTX treatment.**
   **A)** Evolution of the frequencies of follicular autoreactive anti-Dsg3 T cells (Tetramer+CXCR5+) among Ag-experienced T cells (CD4+CD45RA-) in PV patients who received RTX.
   **B)** Study of the impact of the use of RTX on the polarization of Dsg3-specific Tfh cells. Frequencies of each phenotype Tfh1 (red), Tfh1* (green), Tfh2 (pink), Tfh17 (blue) were compared during time between baseline (circle symbols) and LTFU (triangular symbols). Results are presented as frequencies of each polarization according to CXCR3 and CCR6 chemokine receptors, among CD4+CD45RA-Tetramer+CXCR5+ T cells.
   **C)** Comparison of the (Tfh2+Tfh17)/Tfh1 ratio from baseline (circle symbols) to LTFU after RTX (triangular symbols).

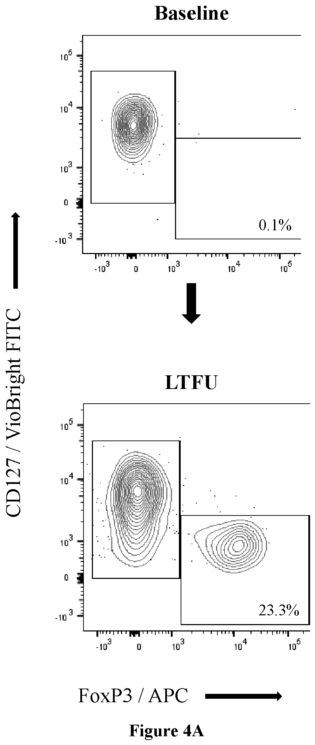

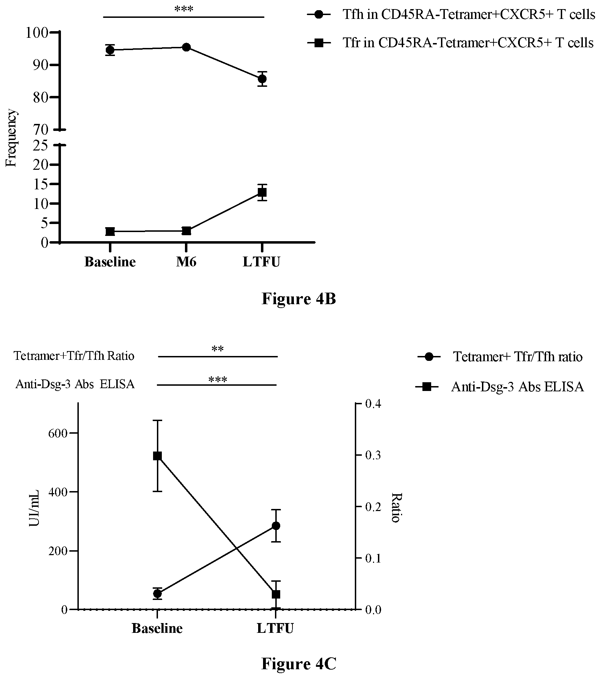

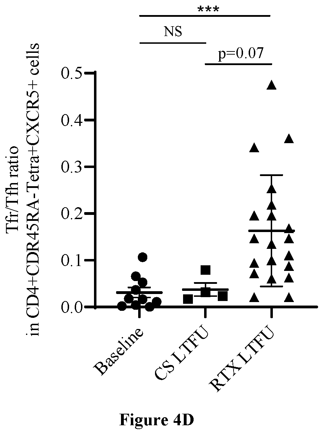

**Figure 4****: Evolution of the Dsg-3-specific Tfr cells after RTX in PV patients.**
   **A)** Example of the course of Dsg3-specific Tfr cells CD4+CD45RA-Tetramer+CXCR5+CD127-FoxP3+ in a patient at the LTFU visit (on the bottom) compared to baseline visit (on the top).
   **B)** Course of the Dsg3-specific Tfh (CXCR5+CD127+FoxP3-, circle symbols) and Tfr (CXCR5+CD127-FoxP3+, triangular symbols) cells after RTX from baseline to Month 6 (M6) and the last LTFU evaluation.
   **C)** Course of the anti-Dsg3 Abs ELISA (square symbols) concomitantly to anti-Dsg3 Tfr/Tfh ratio between baseline and the last post-RTX LTFU evaluation. *** P < 0.001
   **D)** Dsg3-specific Tfr/Tfh ratio in treated PV patients at LTFU versus baseline (circle symbols), according to the first-line treatment used: RTX (black triangular symbols) or CS alone (square symbols). *** P < 0.001
**Figure 5****: Activation of autoreactive Dsg3-specific Tfr cells compared to Dsg-3-specific Tfh and non-autoreactice Tfr and Tfh cells in PV patients after RTX.**

Activation was measured as geometric mean fluorescence intensities (gMFI) of ICOS **(5A),** HLA-DR **(5B)** and CD25 **(5C)** expressions by flow cytometry. gMFI were compared between Tfr Tetramer+ (CD4+CD45RA-Tetramer+CXCR5+CD127-FoxP3+), Tfh Tetramer+ (CD4+CD45RA-Tetramer+CXCR5+CD127+FoxP3-), Tfr Tetramer- (CD4+CD45RA-Tetramer-CXCR5+CD127-FoxP3+) and Tfh Tetramer- (CD4+CD45RA-Tetramer-CXCR5+CD127+FoxP3-) at the M60-M90 visit.

An example of representative contour plots comparing gMFI expression between autoreactive Tfr cells and autoreactive Tfh cells from the PV11 patient is proposed for each activity marker at LTFU: ICOS **(5A),** HLA-DR **(5B)** and CD25 **(5C).**
***P < 0.001; ****P< 0.0001;

### EXAMPLE:

### Material & Methods

### Patients

A total of 90 patients, 74 with pemphigus vulgaris and 16 with pemphigus foliaceus, were included in the original randomized clinical trial (Ritux3; ClinicalTrials.gov number, NCT00784589). The Ethics Committee (CPP Nord-Ouest1) approved the study. Patients with newly diagnosed pemphigus were assigned to receive either 1 or 1.5 mg/kg/day of oral prednisone, with a progressive tapering of prednisone doses over M12 or M18, or two infusions of 1 g of RTX on day 0 and day 14, and 500 mg at M12 and M18, combined with a short-term prednisone regimen, 0.5 mg/kg/day for moderate pemphigus and 1 mg/kg/day for severe pemphigus. The initial prednisone dose was gradually reduced after achievement of disease control, with the aim to stop prednisone after M3 in patients with moderate pemphigus and after M6 in patients with severe pemphigus. Patients were initially followed 36 months and an extension study was approved by ethic committees to assess the long-term clinical and immunological status of patients. Because inclusion period of the Ritux 3 study lasted almost 3 years, this extension visit allowed to record data from 5 years (M60) for the last included patients, to almost 8 years for the first included patients (M90) in Ritux 3. Thus, this visit will be called LTFU.

We retrospectively analysed PBMC from blood samples performed during the follow-up visits initially scheduled in the Ritux 3 trial and also from the last extension visit LTFU. A large part of these samples were already used for previous studies (3,4,20-22). Consequently, we studied blood samples from baseline, M6, M36 and LTFU visits. We had no blood samples from the M12 and M24 visits. Due to the very few available samples at M36, statistical analyses were not feasible and related data are not presented. Overall, data related to the baseline, M6 and LTFU visits are presented.

The study on the PBMC from healthy adult subjects was approved by the French South-West & Overseas ethical committee and was registered at the French Ministry of Higher Education and Research (DC-2015-2488). Experiments were performed in agreement with the guidelines of the Declaration of Helsinki. PBMC were isolated from blood samples provided by the French National Blood Service (EFS) from age- and sex-matched subjects.

### Clinical and immunological evaluations

Complete remission and relapse were defined according to the consensus statement definition for pemphigus end points (23). Participants gave their written informed consent. Blood samples of patients were collected at baseline and at each follow-up visit until the end of the study at the LTFU visit between M60 and M90.

### Serum autoantibody titers

Titers of serum anti-Dsg1 and anti-Dsg3 IgG Abs were measured using an ELISA kit as described by the provider (EUROIMMUN, Lübeck, Germany).

### HLA-genotype testing

To verify that our patients with pemphigus had the HLA-DRB1*0402 genotype of PV susceptibility, peripheral blood from patients was collected after obtaining informed consent. Genomic DNA from PBMCs was isolated using the NucleoSpin tissue kit (Macherey-Nagel, Duren, Germany). The specific analysis of HLA-DRB1*0402 was performed using the Olerup SSP DRB1*04 PCR Typing Kit (Bionobis, Guyancourt, France) according to the manufacturer's protocol. HLA-DRB1*04 was found in 36 out the 90 patients. Autoreactive Tfh and Tfr cells were only characterized in patients with PV with the HLA-DRB 1*0402 genotype.

### Anti-Dsg-3 Tfh and Tfr cell stainings

Frequency and phenotype of circulating Dsg-3-specific Tfh and Tfr cells were determined by flow cytometry using mAbs against CD3 (clone BW264/56), CD4 (VIT4), CD45RA (REA562), CXCR5 (REA103), CD127 (REA614) and FoxP3 (REA1253) (Miltenyi Biotec and BD Biosciences) and a tetramer-based detection system using HLA-DRB 1 *0402-tetramers loaded with the Dsg-3 190-204 immunodominant peptide (LNSKIAFKIVSQEPA, obtained from NIH Tetramer core facility, Emory University, Atlanta, GA) (4). An irrelevant HLA-DRB1*0402-tetramer (human CLIP 87-101 PVSKMRMATPLLMQA, NIH Tetramer core facility, Emory University, Atlanta, GA) was used as negative control for the cytometric gating strategy. The percentages of circulating Dsg-3-specific Tfh and Tfr cells were determined using FlowJo software. Only blood samples allowing to assess more than 100 Dsg3-specific follicular T cells (CD4+CD45RA-Tetramer+CXCR5+) were analyzed.

The frequencies of different phenotypes of circulating follicular helper T cells (Tfh1, Tfh1*, Tfh2, Tfh17) were determined by flow cytometry using mAbs against CCR6 (REA190) and CXCR3 (REA232) (Miltenyi Biotec). Each polarisation was defined as following: Tfh1:CXCR3+CCR6-; Tfh1*: CXCR3+CCR6+; Tfh17: CXCR3-CCR6+; Tfh2: CXCR3-CCR6-.

### Tfh and Tfr cell activation

Circulating Tfh and Tfr cell activation was assessed by the mean fluorescence intensity (gMFI) in flow cytometry using mAbs against CD25 (MA251), HLA-DR (AC122) and ICOS (REA192) (Miltenyi Biotec and BD Biosciences).

### Statistical analysis

Data are presented as mean±SEM. Prism software (Graph Pad, San Diego, CA) was used for statistical analysis. For multiple analyses, one-way ANOVA with Dunnett post-test or two-way ANOVA with Sidak's multiple comparisons test were used. A P < 0.05 was considered significant for all analyses.

### Results

### Clinical course of the studied PV patients

All patients were included in the "Ritux 3" trial (3), which enrolled 90 patients with a newly-diagnosed pemphigus, 46 of whom were initially randomly assigned to receive RTX in combination with a short-term oral regimen of corticosteroids (CS) (oral prednisone 0.5 to 1.0 mg/kg per day tapered over 3 to 6 months = RTX group) and the remaining ones received CS alone (oral prednisone 1.0 to 1.5 mg/kg per day tapered over 12 to 18 months = CS group). At the Month-36 (M36) evaluation, 44 of 46 patients (96%) from the RTX group were in complete remission off therapy, including two patients who received additional RTX infusions between M24 and M36. Two patients had persistent disease activity. An extension visit was secondarily performed, allowing a long-term follow-up (LTFU) of patients between Month 60 and Month 90, according to their initial inclusion date.

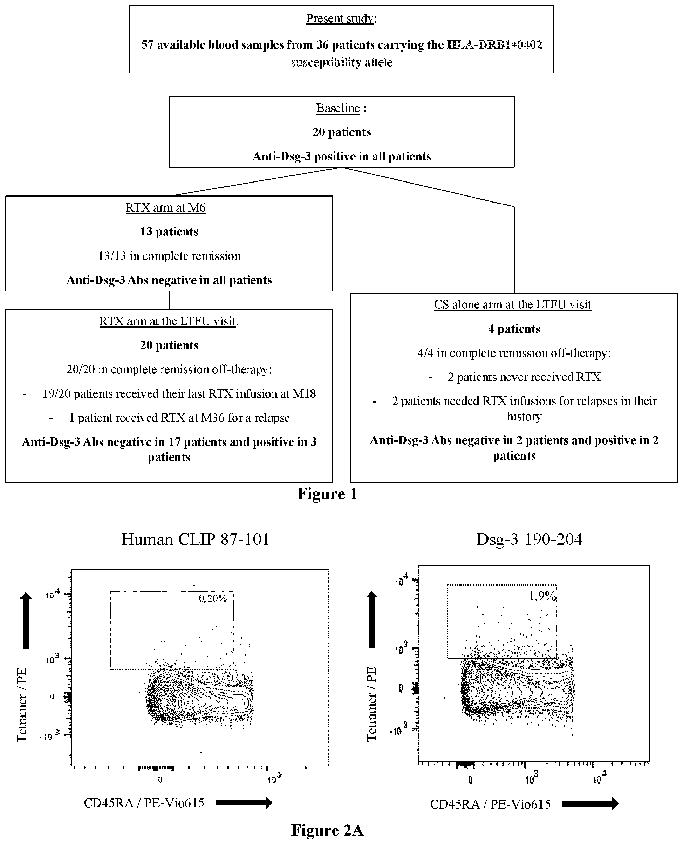

Notably, 36 out of the 90 patients carried the PV susceptibility allele HLA-DRB1*0402. Among them, we had 57 stored blood samples corresponding to different timepoints thus we did not have a longitudinal follow-up for all patients. Each blood sample corresponded to one patient at one time. (Figure 1). This material included 20 blood samples collected at baseline, 13 at M6, and 24 samples collected at the LTFU visit. These latter blood samples included 20 samples from patients treated with RTX, and 4 from patients initially treated with CS-alone. All patients were in complete remission off therapy at this LTFU visit. Among the 20 patients from the RTX arm whose blood samples were assessed at the LTFU visit, only one patient has received two additional RTX infusions since M18 (date of the last scheduled RTX infusion according to the protocol). Interestingly, 7 patients from the RTX arm had a longitudinal follow-up with available blood samples for the baseline, M6 and LTFU visits. Among the 4 patients initially enrolled in the CS group, 2 needed onset of RTX infusions at M50 and M70, respectively.

### Long-term assessment of serum anti-Dsg-3Abs after RTX treatment

Mean ELISA value of anti-Dsg3 Abs decreased from 522.5±381.5 IU/mL at baseline to 51.9±189.0 IU/mL in serum samples collected at the LTFU visit (p<0.001). From the end of the Ritux 3 trial at M36 to the LTFU evaluation in the present study, out of the 20 patients initially assigned in the RTX group, anti-Dsg-3 auto-Ab ELISA values remained negative in 17 whereas they reappeared in 3 patients (997, 785 and 405 IU/mL, respectively) despite they maintained a sustained clinical complete remission.

Out of the 4 patients initially treated by CS alone, two patients had persistent positive anti-Dsg-3 Ab ELISA values at the LTFU visit (42 and 305 IU/mL) and relapsed between M36 and the LFTU visit, corresponding to the two patients who needed RTX infusions. The two other patients remained in sustained complete remission and did not exhibit any anti-Dsg-3 Abs during their follow-up.

### Baseline phenotype of Dsg-3-specific follicular T cells in PV patients

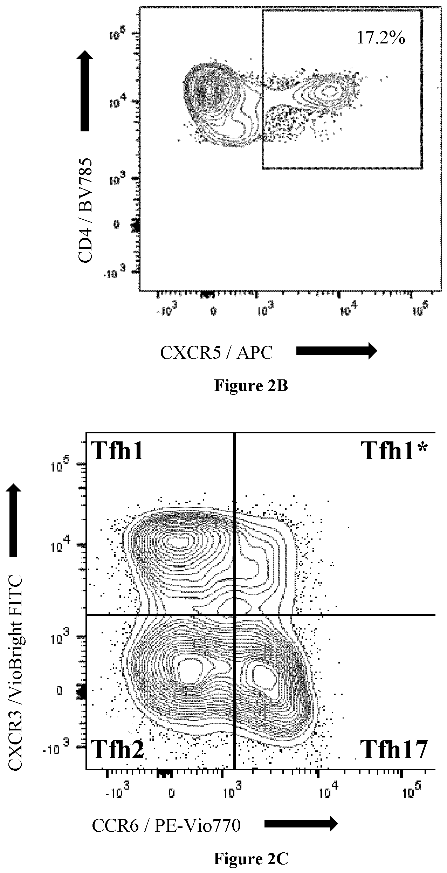

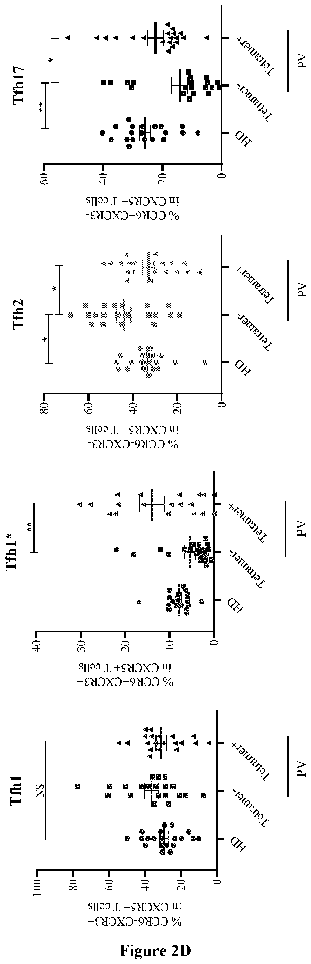

Using the tetramer HLA-DRB1*0402 loaded with the immunodominant peptide of Dsg-3 (190-204) and an irrelevant control (human CLIP 87-101), we were able to detect Dsg-3-specific T cells (Figure 2A). Then, we assessed the baseline polarisation (Tfh1 (CXCR3+CCR6-), Tfh1* (CXCR3+CCR6+), Tfh2 (CXCR3-CCR6-), Tfh17 (CXCR3-CCR6+)) of circulating Dsg-3-specific follicular T cells (CD4+CD45RA-Tetramer+CXCR5+) based on the expression of the chemokine receptors CXCR5 (Figure 2B), CXCR3 and CCR6 (Figure 2C), and compared these proportions to: i) those on circulating non-autoreactive follicular T cells (CD4+CD45RA-Tetramer-CXCR5+) from PV patients and ii) those measured in circulating follicular T cells (CD4+CD45RA-CXCR5+) from 20 healthy age- and sex-matched donors (HD). The baseline frequency of circulating autoreactive Dsg-3 specific Tfh1 cells was not different from that observed in non-autoreactive Tfh1 cells from PV patients nor from HD. Interestingly, Tfh1* and Tfh17 subsets were over-represented in Dsg-3-specific CXCR5+ T cells relative to non-autoreactive CXCR5+ T cells from PV patients (mean±SEM: Tfh1*: 13.94±2.69% vs 5.42±1.3%; p= 0.004; Tfh17: 22.32±2.59% vs 14.09±2.8%; p= 0.046) (Figure 2D). In contrast, the Tfh2 subset was under-represented in autoreactive compared to circulating non-autoreactive follicular T cells from PV patients (32.91±2.72% vs 44.09±3.2%; p= 0.014) (Figure 2D). Overall, the polarisation of circulating autoreactive Dsg-3-specific Tfh cells was skewed toward Tfh17 and was clearly different from that observed in non-autoreactive Tfh cells from the same PV patients but quite similar to that observed in non-autoreactive Tfh cells from HDs.

### Long-term evolution of circulating Dsg-3-specific follicular T cell polarisation after RTX treatment

We then studied the course of circulating autoreactive Dsg-3-specific follicular T cells by flow cytometry, from baseline to the LTFU evaluation. Despite the fact that we initially observed a decrease in the frequency of Dsg-3-specific follicular T cells (CD4+CD45RA-Tetramer+CXCR5+) between baseline and M36 (4), we showed in the present study that the mean frequency of circulating Dsg-3-specific follicular T cells measured at the M60-M90 evaluation was closely similar to the one measured at baseline (mean±SEM: baseline: 0.47±0.05% vs M60-M90: 0.53±0.05% ; p=0.24) (Figure 3A).

Since we found that the frequencies of circulating Dsg-3-specific follicular T cells at the LTFU evaluation were close to those observed at baseline, we tested the hypothesis that a change in their polarisation might explain the blockage of the Ig class switch of autoreactive B cells observed in PV patients in sustained clinical remission after RTX treatment. For this, we studied the polarisation of autoreactive Dsg-3-specific CXCR5+ T cells at the LTFU evaluation i.e. several years after the last M36 infusion of RTX scheduled in the study protocol (Figure 3B). We did not observe any significant change in the respective proportions of Tfh1, Tfh1*, Tfh2 or Tfh17 of circulating Dsg-3-specific CXCR5+ T cells from baseline to the long follow-up times.

It has been demonstrated in other Ab-mediated autoimmune diseases such as juvenile dermatomyositis, Sjogren syndrome or systemic lupus erythematous that the (Tfh2+Tfh17)/Tfh1 ratio of blood circulating CXCR5+ T cells was significantly increased (> 1) during the active phase of the disease, and around 1 in patients in complete remission (24). Similarly, the baseline (Tfh2+Tfh17)/Tfh1 ratio of Dsg-3-specific CXCR5+ T cells of PV patients was higher than 1 (mean±SEM: 2.98:1:1.01;) (Figure 3C). Notably, this ratio remained significantly higher than 1 on the long term (mean±SEM: LTFU: 2.75±0.41; p=0.43 versus baseline). Thus, while RTX treatment induces complete remission in PV patients, it does not impact Dsg-3-specific follicular T cell polarisation.

### Emergence of circulating Dsg-3-specific regulatory follicular T (Tfr) cells after RTX treatment

Since we did not observe major changes in the number and polarisation of circulating Dsg-3-specific CXCR5+ T cells which could explain the long-term efficacy of RTX in PV patients, we then studied the effect of RTX on circulating Dsg-3-specific Tfr cells. Interestingly, we observed that the frequency of circulating Tfr cells (Tetramer+CXCR5+CD127-FoxP3+) were almost absent at baseline and increased lately after RTX since their frequencies measured at baseline and M6 were low and significantly increased at LTFU (mean±SEM: 2.80%±0.92 at baseline, 3.00% ±0.80 at M6, 12.85%±2.06 at LTFU; p<0.001) (Figure 4A and 4B). This change in the ratio between Tfr and Tfh going from 0.03±0.01 at baseline to 0.16±0.03 at the LTFU evaluation; (p<0.005) correlated to a prolonged decrease of anti-Dsg3 auto-Abs, even many years after the start of treatment, while no infusion of RTX was performed since the M1 8 visit (Figure 4C).

These results are in accordance with our previous findings (4), in which we observed a decrease in the frequency of bona-fide Tfh cells (FOXP3-), among circulating follicular Dsg-3-specific T cells (Tetramer+CXCR5+); initially stable during the first months after RTX treatment (mean±SEM: 94.61±1.59% at baseline versus 95.50±0.81% at M6), and then decreased at LTFU (85.68±2.20% at LTFU; p<0.001) (Figure 4B). In the 7 patients with longitudinal follow-up, all had an increase of their Tfr frequencies at the LTFU visit compared to baseline and M6 visits (data not shown).

Interestingly, we also could analyze blood samples from the four patients who were initially treated with CS alone. Two of them never received RTX whereas the two others lately received RTX in their disease history, that means relatively closely from the LTFU visit. We showed that the Tfr/Tfh ratio in circulating Dsg-3-specific follicular T cells from the LTFU samples in these patients was closely similar to the one observed at baseline in PV patients while the one observed in patients initially treated with RTX was significantly higher (Figure 4D).

### Late overactivation of autoreactive regulatory T cells

We then evaluated the activation status of the Tfr cell sub-population by comparing the membrane expression (gMFI) of ICOS, HLA-DR and CD25 at the surface of circulating Tfr and Tfh cells either Dsg-3-specific or not. Due to the very small frequencies of Dsg-3-specific Tfr cells at baseline and Month 6, the activation could only be assessed at LTFU. We observed that Dsg-3-specific Tfr cells were significantly overactivated as compared to Dsg-3-specific Tfh cells (Figure 5). Additionally, the high expression of ICOS, HLA-DR and CD25 was only observed on circulating Dsg-3-specific Tfr cells, but not on non-autoreactive Tfr cells from the same PV patients, suggesting that this over-activation was specific for autoreactive Dsg-3-specific Tfr cells. Finally, we found similar results in non-CXCR5+ regulatory T cells which were overactivated compared to autoreactive Th cells as well as non-autoreactive Treg and Th cells (data not shown).

### Conclusion

Overall, the major findings from this study are: i) the in vivo highlighting of a circulating autoreactive Tfr cell population in PV after RTX treatment and ii) its role in the long-term clinical remission after treatment with RTX.

### REFERENCES:

Throughout this application, various references describe the state of the art to which this invention pertains. The disclosures of these references are hereby incorporated by reference into the present disclosure.
1. Kasperkiewicz M, Ellebrecht CT, Takahashi H, Yamagami J, Zillikens D, Payne AS, et al. Pemphigus. Nat Rev Dis Primer. 11 mai 2017;3:17026.
2. Schmidt E, Kasperkiewicz M, Joly P. Pemphigus. The Lancet. 7 sept 2019;394(10201):882-94.
3. Joly P, Maho-Vaillant M, Prost-Squarcioni C, Hebert V, Houivet E, Calbo S, et al. First-line rituximab combined with short-term prednisone versus prednisone alone for the treatment of pemphigus (Ritux 3): a prospective, multicentre, parallel-group, open-label randomised trial. Lancet Lond Engl. 20 mai 2017;389(10083):2031-40.
4. Maho-Vaillant M, Perals C, Golinski ML, Hébert V, Caillot F, Mignard C, et al. Rituximab and Corticosteroid Effect on Desmoglein-Specific B Cells and Desmoglein-Specific T Follicular Helper Cells in Pemphigus. J Invest Dermatol. 22 mars 2021;
5. Holstein J, Solimani F, Baum C, Meier K, Pollmann R, Didona D, et al. Immunophenotyping in pemphigus reveals a TH17/TFH17 cell-dominated immune response promoting desmoglein1/3-specific autoantibody production. J Allergy Clin Immunol. juin 2021;147(6):2358-69.
6. Zou Y, Yuan H, Zhou S, Zhou Y, Zheng J, Zhu H, et al. The Pathogenic Role of CD4+ Tissue-Resident Memory T Cells Bearing T Follicular Helper-Like Phenotype in Pemphigus Lesions. J Invest Dermatol. 1 sept 2021;141(9):2141-50.
7. Takahashi H, Iriki H, Asahina Y. T cell autoimmunity and immune regulation to desmoglein 3, a pemphigus autoantigen. J Dermatol. 2023;50(2):112-23.
8. Kim AR, Han D, Choi JY, Seok J, Kim SE, Seo SH, et al. Targeting inducible costimulator expressed on CXCR5+PD-1+ TH cells suppresses the progression of pemphigus vulgaris. J Allergy Clin Immunol. nov 2020;146(5):1070-1079.e8.
9. Fazilleau N, McHeyzer-Williams LJ, Rosen H, McHeyzer-Williams MG. The function of follicular helper T cells is regulated by the strength of T cell antigen receptor binding. Nat Immunol. avr 2009;10(4):375-84.
10. Victora GD, Nussenzweig MC. Germinal Centers. Annu Rev Immunol. 23 avr 2012;30(1):429-57.
11. Chung Y, Tanaka S, Chu F, Nurieva RI, Martinez GJ, Rawal S, et al. Follicular regulatory T cells expressing Foxp3 and Bcl-6 suppress germinal center reactions. Nat Med. 24 juill 2011;17(8):983-8.
12. Linterman MA, Pierson W, Lee SK, Kallies A, Kawamoto S, Rayner TF, et al. Foxp3+ follicular regulatory T cells control the germinal center response. Nat Med. 24 juill 2011;17(8):975-82.
13. Wollenberg I, Agua-Doce A, Hernández A, Almeida C, Oliveira VG, Faro J, et al. Regulation of the germinal center reaction by Foxp3+ follicular regulatory T cells. J Immunol Baltim Md 1950. 1 nov 2011;187(9):4553-60.
14. Sage PT, Paterson AM, Lovitch SB, Sharpe AH. The Coinhibitory Receptor CTLA-4 Controls B Cell Responses by Modulating T Follicular Helper, T Follicular Regulatory, and T Regulatory Cells. Immunity. 18 déc 2014;41(6):1026-39.
15. Wing JB, Ise W, Kurosaki T, Sakaguchi S. Regulatory T Cells Control Antigen-Specific Expansion of Tfh Cell Number and Humoral Immune Responses via the Coreceptor CTLA-4. Immunity. 18 déc 2014;41(6):1013-25.
16. Clement RL, Daccache J, Mohammed MT, Diallo A, Blazar BR, Kuchroo VK, et al. Follicular regulatory T cells control humoral and allergic immunity by restraining early B cell responses. Nat Immunol. oct 2019;20(10):1360-71.
17. Sage PT, Ron-Harel N, Juneja VR, Sen DR, Maleri S, Sungnak W, et al. Suppression by T FR cells leads to durable and selective inhibition of B cell effector function. Nat Immunol. déc 2016;17(12):1436-46.
18. Botta D, Fuller MJ, Marquez-Lago TT, Bachus H, Bradley JE, Weinmann AS, et al. Dynamic regulation of T follicular regulatory cell responses by interleukin 2 during influenza infection. Nat Immunol. nov 2017;18(11):1249-60.
19. Fu W, Liu X, Lin X, Feng H, Sun L, Li S, et al. Deficiency in T follicular regulatory cells promotes autoimmunity. J Exp Med. 29 janv 2018;215(3):815-25.
20. Hébert V, Petit M, Maho-Vaillant M, Golinski ML, Riou G, Derambure C, et al. Modifications of the Transcriptomic Profile of Autoreactive B Cells From Pemphigus Patients After Treatment With Rituximab or a Standard Corticosteroid Regimen. Front Immunol. 2019;10:1794.
21. Hébert V, Maho-Vaillant M, Golinski ML, Petit M, Riou G, Boyer O, et al. Modifications of the BAFF/BAFF-Receptor Axis in Patients With Pemphigus Treated With Rituximab Versus Standard Corticosteroid Regimen. Front Immunol [Internet]. 2021 [cité 17 mai 2021];12. Disponible sur: https://www.frontiersin.org/articles/10.3389/fimmu.2021.666022/full?&utm_source=Email_t o_authors_&utm_medium=Email&utm_content=T1_11.5e1_author&utm_campaign=Email_ publication&field=&journalName=Frontiers_in_Immunology&id=666022
22. Golinski ML, Lemieux A, Maho-Vaillant M, Barray M, Drouot L, Schapman D, et al. The Diversity of Serum Anti-DSG3 IgG Subclasses Has a Major Impact on Pemphigus Activity and Is Predictive of Relapses After Treatment With Rituximab. Front Immunol [Internet]. 2022 [cité 28 mars 2022];13. Disponible sur: https://www.frontiersin.org/article/10.3389/fimmu.2022.849790
23. Murrell DF, Dick S, Ahmed AR, Amagai M, Barnadas MA, Borradori L, et al. Consensus statement on definitions of disease, end points, and therapeutic response for pemphigus. J Am Acad Dermatol. juin 2008;58(6):1043-6.
24. Morita R, Schmitt N, Bentebibel SE, Ranganathan R, Bourdery L, Zurawski G, et al. Human blood CXCR5(+)CD4(+) T cells are counterparts of T follicular cells and contain specific subsets that differentially support antibody secretion. Immunity. 28 janv 2011;34(1):108-21.

## Claims

1. A method of determining whether a patient suffering from a Pemphigus vulgaris (PV) has or is at risk of relapsing after a rituximab (RTX) treatment comprising the steps of: i) determining in a sample obtained from said patient the quantity of Dsg-3-Tfr cells ii) comparing said quantity determined at step i) with a predetermined reference value and iii) providing that a high quantity of Dsg-3-Tfr cells compared to said predetermined reference value is indicative of whether said patient has not or is not at risk of relapsing and providing that absence or a low quantity of Dsg-3-Tfr cells compared to said predetermined reference value is indicative of whether said patient has or is at risk of relapsing.

2. A method of determining whether a patient suffering from a Pemphigus vulgaris (PV) has or is at risk of relapsing after a rituximab (RTX) treatment comprising the steps of: i) determining in a sample obtained from the patient the quantity of Dsg-3-Tfr cells and Dsg-3-Tfh cells ii) calculating the Tfr/Tfh ratio and iii) providing that a ratio < 1 is indicative of whether a patient has or is at risk of relapsing and that a ratio > or = 1 is indicative of whether a patient has not or is not at risk of relapsing.

3. The method according to the claims 1 or 2 wherein the Dsg-3-Tfr cells are autoreactive Dsg-3-Tfr cells.

4. The method according to the claims 1 to 3 wherein the determination of the quantity of Dsg-3-Tfr cells or Dsg-3-Tfh cells is realized between 6 months and 90 months after the beginning of the treatment with RTX.

5. The method according to the claims 1 to 4 wherein the sample can be blood and peripheral-blood.

6. A method for treating a Pemphigus vulgaris in a patient which is relapsing or will relapse after a RTX treatment as determined with the methods of claims 1 or 2 comprising the administration to said patient of corticosteroid treatment.
